# EUROPEAN PATENT SPECIFICATION

(11) **EP 2 696 853 B1**
(45) Date of publication and mention of the grant of the patent: **23.10.2019**
(21) Application number: 12723933.3
(22) Date of filing: 11.04.2012
(51) Int. Cl.: A61K 9/127, A61K 47/34

(54) **SURFACE INDUCED DISASSEMBLY OF NANO CONTAINERS**
OBERFLÄCHENINDUZIERTE DEMONTAGE VON NANOBEHÄLTER
DISSOCIATION DE NANO-CONTAINERS INDUITE EN SURFACE

(30) Priority: 11.04.2011 IN 1033DE2011
(43) Date of publication of application: 19.02.2014
(73) Proprietor: Council of Scientific & Industrial Research, New Delhi 110001 (IN)
(72) Inventor: KRISHNAMOORTHY, Kothandam, Pune 411008 Maharashtra (IN)
(74) Representative: Connell, Mark
(86) International application number: PCT/IN2012/000259
(87) International publication number: WO 2012/140674

(56) References cited:
- WO-A1-2004/083282
- US-A1- 2007 082 246
- US-B1- 6 569 651
- P. A. HASSAN ET AL: "Polyaniline Nanoparticles Prepared in Rodlike Micelles", LANGMUIR, vol. 20, no. 12, 1 June 2004 (2004-06-01), pages 4874-4880, XP55034059, ISSN: 0743-7463, DOI: 10.1021/la0498096 cited in the application

## Description

### Technical field of invention:

The present invention discloses stimuli induced disassembly of nano containers by charged surface. Particularly, the present invention provides stimuli induced disassembly of nano containers by polyvalent interaction between surfactant assemblies and delocalized charges of an insoluble polymer to trigger the disassembly for site specific delivery of guest molecules.

### Background and prior art:

The nanomaterials based on polymers and amphiphilic self-assembling systems are reported in the literature. The amphiphilic molecules are defined as molecules with exterior hydrophilic end and hydrophobic interior that arrange themselves at interfaces to build aggregates in solution. Further the amphiphiles are surface active molecules; which lower the surface tension, thereby lowering the energy of the system.

Nanoassemblies of amphiphilic molecules are promising candidates for payload encapsulation and delivery at desired locations. Delivery of the payload is initiated by disassembly of nanoassemblies in response to stimulus such as temperature, pH, magnetic field, ultrasound, redox, proteins and enzymes.

The polymer-micelle complex has standard ability to tune the core and shell components, such complexes particularly useful for drug delivery because the core of the assembly can serve as a reservoir for a variety of therapeutic agents while the hydrophilic shell imparts solubility and stability to the aqueous assemblies.

Polymer-micelles are vital due to their ability to deliver large payloads of a variety of drugs (e.g. biomolecules, dyes, drugs, proteins, enzymes and DNA/RNA therapeutics), and their improved in vivo stability as compared to other colloidal carriers (e.g. liposomes), and their nanoscopic size which allows for passive accumulation in diseased tissues, such as solid tumors, by the enhanced permeation and retention (EPR) effect. The polymer micelle complex can actively target diseased cells and aid in cellular entry, resulting in improved cell specific delivery.

United States Patent Application No. 20090081457 discloses a polymer-micelle complex suitable for use as an aid to preparing fibers, particularly nanofibers, by electrospinning wherein the polymer is selected from the group consisting of polyethylene oxide (PEO); polyvinyl pyrrolidone (PVP); polyvinyl alcohol (PVOH); polyamides (PA); polyacrylonitrile (PAN); polyaniline (PANI)-PEO; PANI/polystyrene (PS); the material may be contained within the micelle, itself, which may be desirable if the material is a drug, an enzyme or other material whose activity one wishes to control and the surfactants may include ionic surfactants and nonionic surfactants depending upon the type of solvent and the type of polymer.

US7638558 relate to a drug-loaded micelle comprising a tri-block copolymer, wherein said micelle has a drug-loaded inner core, a crosslinked outer core, and a hydrophilic shell, wherein the multi-block copolymer comprises a polymeric hydrophilic block, a crosslinked poly (amino acid block), and a poly (amino acid block), characterized in that said micelle has an inner core, a crosslinked outer core, and a hydrophilic shell.

Applications of electrospun fibers in drug delivery, wherein both degradable and non-degradable polymers are to be developed as drug carriers for local delivery of antibiotics and anticancer drugs, by using electrospun fibrous materials with different structures are disclosed in Sci. Technol. Adv. Mater by Wenguo Cui et al. 11 (2010) 014108 (11pp).

Nanocontainers, such as *Tobacco mosaic virus,* zeolites, biological vesicles, polymeric nanogels, solid lipid nanoparticles, or self-assembled peptides, that protect the drug from enzymatic degradation and are sensitive to external stimuli, e.g. heat and pH change, which control the release of chemotherapeutic drugs are disclosed in 'Magnetic Nanocontainers for Combined Hyperthermia and Controlled Drug Release Project' Articles.

United States Patent Application No.20110229565 discloses a drug-delivery composition comprising a drug-derived or prodrug-derived gelator which can be released upon a stimulus wherein stimuli can be ultrasound, temperature, pH, metal ions, light, electrical stimuli, electromagnetic stimuli, and combinations thereof. It also discloses dye encapsulated self-assembled nanofibers, in vitro and in vivo analysis.

Technical report, Polymer Engineering and Science by Wu, Tzong-Ming et al, 04,2008;(48)(40) discloses preparation of doped polyaniline in its emeraldine salt form (PANI-ES) by in situ chemical oxidative polymerization using various concentrations of sodium dodecyl sulfate (SDS) micellar solutions and the synthesis, processing and applications of polyaniline (PANI) conducting polymer, over the other conducting polymers (ICPs) is reported in 'Progress in Polymer Science' 34(8),08-2009 (783-810) by Sambhu Bhadraa et al.

The effect of polyaniline on the size and shape of sodium dodecyl sulfate (SDS) micelles is disclosed in Langmuir, 2004, 20 (12) (4874-4880) and Chemistry Letters Vol. 37 (8) 858; 2008.Further enzyme, protein triggered disassembly of dendrimer-based amphiphilic nanocontainers reported in J. Am. Chem. Soc by Azagarsamy, 2009, 131 (40) (14184-14185).

Additionally, Yajuy n Wang in Phys. Chem. Chem. Phys, 2011, (13),4782-4801 discloses nanostructured polymer assemblies formed at interfaces to generate materials with specific properties and functions for application in diverse fields, including biomaterials, drug delivery, catalysis, sensing, optics and corrosion.

In view of above, it is clear that the delivery of an effective concentration of drug at the right time and at the right place can minimize systemic adverse reactions, reduce drug dosage and eventually lower the cost and improve therapeutic outcome and patient compliance. Research into stimuli-responsive polymers as a means of achieving this is steadily gaining momentum and more novel polymers are being synthesized to be incorporated into innovative delivery systems intended for site-specific and self-regulated drug delivery. These stimuli-responsive polymers are also immensely valuable in applications such as bioseparation of proteins and other bio-particles for basic life science research as well as industrial applications.

In all these approaches, the molecules that form the assembly are suitably designed and synthesized to respond to the stimulus, however, fails to teach site specific delivery. Further, many of the existing methods relate to doped conducting polymer assembly or in the form of gelator, where the viscosity of solution is decreased with increasing concentration of the surfactant.

In view of the above, there is still a need to find new composition with enhanced performance over the existing delivery systems facing the demand of industry for increasingly sophisticated systems for site specific delivery of guest molecules. The present research trends in stimuli-responsive systems are focused on the employment of smart polymers such as charge conducting polymer in order to achieve site-specific delivery of guest molecule which relies solely on a stimulus specific to that region

Therefore, the objective of the present invention is to provide stimuli induced nano containers obtained by charged surface disassembly of amphiphilic molecules as promising candidates for payload encapsulation and delivery at desired location.

### Summary of the Invention:

An example relates to a composition for stimuli induced disassembly of nanocontainer useful for site specific delivery comprising insoluble cationic oxidised conducting polymer; micelle assembly and guest molecule; wherein site specific delivery of guest molecule is achieved by polyvalent interaction between micelle assemblies and delocalized charges of polymer to trigger the disassembly.

The cationic oxidised conducting polymer may be selected from polythiophene (PTs), polypyrrole (PPy), and Poly(3,4-ethylenedioxythiophene) poly(styrenesulfonate) (PEDOT/PSS), polyaniline (PANI), polystyrene (PS) in the range of 1-50% by weight of the composition.

The cationic oxidised conducting polymer may be in bulk, nano, film, patch or device form.

The cationic oxidised conducting polymer may be polyaniline (PANI).

The cationic oxidised conducting polymer may be Poly(3,4-ethylenedioxythiophene).

The micelle assembly may comprise ionic micelle at its CMC.

The guest molecule may be a hydrophobic molecule, not more than 1% by weight of the composition.

The guest molecule may be selected from drugs, dyes, small molecules, biomolecules, proteins and like thereof.

The said composition may be recyclable.

In one aspect, the present invention provides a process for preparation of stimuli induced disassembly of nanocontainer comprising of;
a) dissolving ionic micelle in a solvent at its CMC to form a solution of micelle assembly;
b) adding a guest molecule to the solution of step (a); and
c) disassembly of the micelle assembly and concomitant release of the guest molecule by addition of an oxidized conducting polymer.

One of the feature of the invention is wherein said process is recyclable.

The present invention relates to stimuli induced disassembly of nanocontainer useful for site specific delivery comprising insoluble cationic oxidised conducting polymer; micelle assembly and guest molecule; wherein site specific delivery of guest molecule is achieved by polyvalent interaction between micelle assemblies and delocalized charges of polymer to trigger the disassembly.

### Abbreviations:

DLS: Dynamic light Scattering
SDS: Sodium dodecyl sulfate
TEM: Transmission Electron Microscopy
PANI: Polyanilline
(OxCP): Oxidised conducting polymer
(RedCP): Reduced conducting polymer
(NeuCP):Neutral conducting polymer
(CTAB): Cetyl-trimethyl ammonium bromide

### Description of drawings:

Fig 1: Figure 1 depicts interaction between cationic oxidized conducting polymer (OxCP) and anionic micelle.

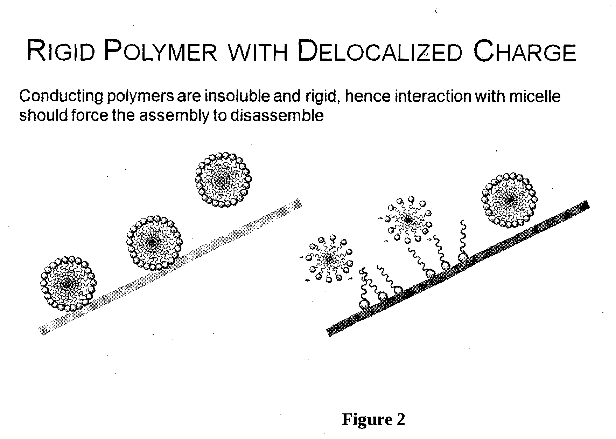

Fig 2: Figure 2 shows interaction between micelle encapsulated with guest molecule and charged, rigid surface of conducting polymer.
Fig 3: depicts DLS of SDS micelle assembly
Fig 4: depicts disassembly of SDS micelle by oxidized PANI
Fig 5: Figure 5 shows interaction of Ox PANI with anionic arid cationic micelles
Fig 6: depicts TEM image of PANI nanofibre
Fig 7: depicts Release of pyrene using PANI and Nanofibre PANI
Fig 8: depicts % release vs number of cycles
Fig 9: Release of guest molecule (Nile red) from micelles using poly(3,4-ethylenedioxythiophene) as a function of time. Decrease in emission intensity as function of time indicates release of guest molecule.
Fig 10: Release of guest molecule (Nile red) from micelles using polyaniline as a function of time. Decrease in emission intensity as function of time indicates release of guest molecule.
Fig 11: Release of guest molecule (Nile red) from DBS micelles using oxidised polyaniline as a function of time. Decrease in emission intensity as function of time indicates release of guest molecule.

### Detailed description of invention:

The present invention describes stimuli (such as temp, pH, ultrasound etc) induced disassembly of nanostructure by using charged polymer surface, wherein nanostructure encapsulated with hydrophobic guest molecule or encapsulant is released at desired location in short time. The nanostructures for targeting delivery of encapsulant are selected from lipid-based liposomes and micelles, protein-based dendrimers and polymer-based nanovectors such as nanoparticles, nanospheres and nanocontainers.

In an embodiment, present invention provides a process for stimuli induced disassembly of nanocontainer useful for site specific delivery comprising insoluble cationic oxidised conducting polymer; micelle assembly and guest molecule; wherein site specific delivery of guest molecule is achieved by polyvalent interaction between micelle assemblies and delocalized charges of polymer to trigger the disassembly. Insoluble cationic oxidised conducting polymer of the invention is insoluble in the micelle solution.

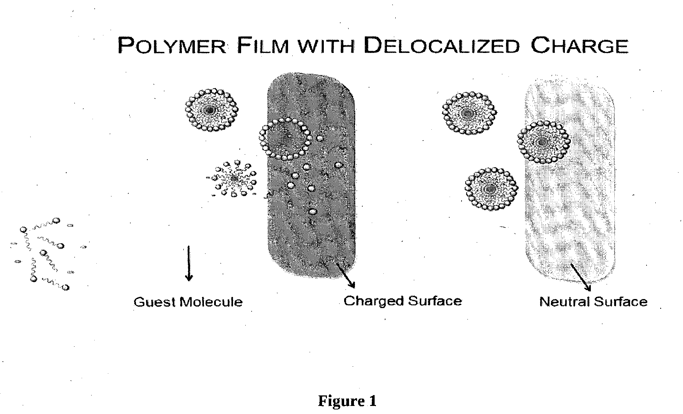

Accordingly the present inventors evaluated the interaction between insoluble cationic oxidized conducting polymer (OxCP) and ionic micelle using DLS, wherein the micelle assembly size is reduced from 6 nm to 1 nm due to polyvalent interaction, on the other hand no anionic micelle disassembly is observed in presence of neutral conducting polymer (NeuCP) as shown in figure 1.

In an embodiment the insoluble cationic oxidized conducting polymer is selected from the group comprising of polythiophene (PTs), polypyrrole (PPy), and Poly(3,4-ethylenedioxythiophene):poly(styrenesulfonate) PEDOT/PSS. polyaniline (PANI), polystyrene (PS), wherein polyaniline is especially attractive among them because it is less expensive, has three distinct oxidation states with different colors and has good acid/base doping response. The polymer is used in the composition in the range of 1-50 by weight of composition.

The nanoassembly comprises of ionic micelles including cationic surfactants, such as cetyl-trimethyl ammonium bromide (CTAB) and dodecyl trimethylammonium bromide (DTAB) and anionic surfactant such as do decyl benznene sulphoneate (DBS) and sodium dodecyl sulfate (SDS) with known critical micelle concentrations (CMC) and surfactant aggregation numbers, preferably anionic micelle sodium dodecyl sulfate (SDS). The ionic micelle is used at its CMC.

In yet another embodiment, the guest molecule is hydrophobic selected from drug, dye, small molecules, biomolecule, protein and like thereof. Further the hydrophobic chemotherapeutic drug is selected from anticancer agent, antihistamic agent, antibacterial agent, antifungal agent, CNS, cardiovascular, antibiotic agent, NSAID and anti-inflammatory agent. The guest molecule is used in the nano container of the invention at not more than 1% by weight of composition.

In yet another embodiment, the process of the invention wherein stimuli induced nanocontainers obtained by charged surface disassembly of amphiphilic molecules are used as candidates for payload encapsulation and delivery at desired locations comprises: oxidized polymer bearing positive charge as surface to induce disassembly of nanoscopic containers having anionic charges on the solvent exposed exterior and hydrophobic interior, and wherein the nanoscopic containers have sequestered hydrophobic guest molecules.

In another embodiment, the present invention provides process for preparation of stimuli induced disassembly of nanocontainer comprising step of;
a) dissolving ionic micelle in solvent at its CMC to form micelle assembly;
b) adding guest molecule to solution of step (a) at a concentration of not more than 1% by weight of the composition; and
c) disassembly of micelle and concomitant release of guest molecule by addition of oxidized conducting polymer at 1-50% by weight of the polymer composition..

The soft template synthesis, also named self-assembly method, employs micelles formed by surfactants to confine the polymerization of conducting polymers into low dimensional nanomaterials.

In yet another embodiment the conducting polymer of the present invention is in the form selected from, bulk form, nano form, film, patch, device and like thereof, wherein the release or delivery of candidates from the composition is independent of the form of the composition.

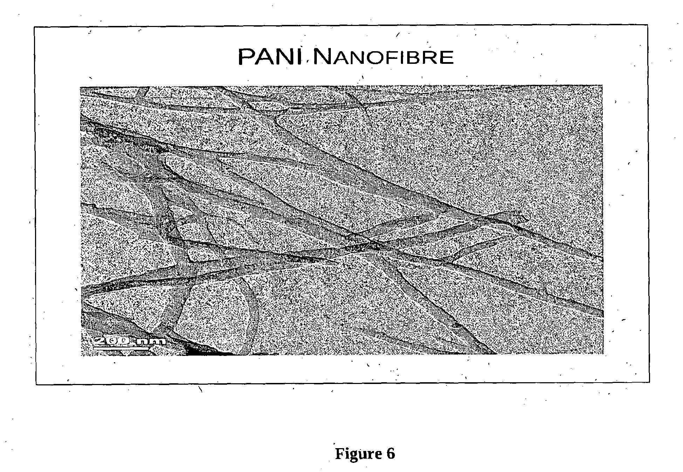

According to the embodiment the oxidizing conducting polymer nanofibres are ranging from 20-200 nm in diameter, particularly nanofibres of PANI (Fig.6), whereas the size of nanoassembly of anionic micelles is in the range of 0.1 to 5 nanometers in diameter. The anionic micelles employed in the present invention having diameter in the range of 2-20 nm.

Further the present process finds various applications such as pharmaceuticals, biological applications and nanotechnology. Compositions may be administered orally, parenterally, by inhalation spray, by nasal aerosol or inhalation, topically, rectally in the form of suppositories, nasally, buccally, vaginally or via an implanted reservoir, wherein parenteral administration includes subcutaneous, intravenous, intramuscular, intra-articular,intra-synovial, intra sternal, intrathecal, intrahepatic, intralesional and intracranial injection or infusion techniques.

The pharmaceutically acceptable compositions may be orally administered in any orally acceptable dosage forms such as to granules, pellets, beads, seeds, capsules, tablets, oral aqueous suspensions, solutions, dry syrup, oral liquids etc. which can be formulated in the immediate or sustained or controlled or delayed release dosage forms or combinations thereof.

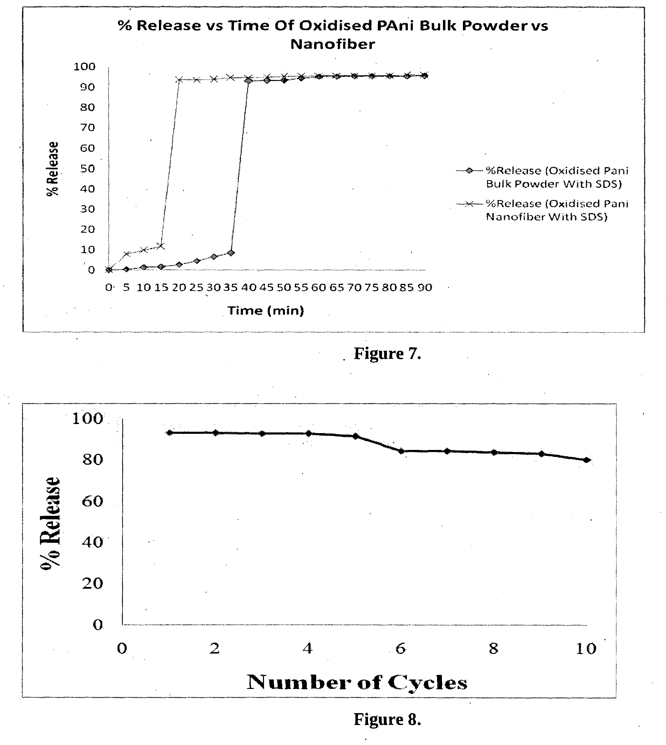

In another embodiment, the conducting polymer surface can be made neutral by reversing the potential therefore one would be able to reuse the substrate for release of guest molecule. Thus the process of the invention is recyclable. With reference to figure 8, the nanoassembly based composition of example 1 is recycled up to 6-10 times wherein the release of guest molecule is more than 50%.

The advantages of nano-sized encapsulation devices are numerous. For example, when compared to single molecule drugs or diagnostic agents, "nanocontainers" can transport much larger quantities of such agents. Nanoscopic drug delivery systems are generally more apt to elude biological barriers, resulting in reduced inactivation or excretion of the encapsulated therapeutic. Multi- functionality is a common feature of nanovectors whereby multiple drugs, diagnostic agents, and targeting groups can be packaged into a single system. The bottom-up design of nanoscopic drug delivery systems often
involves the precise self-assembly of single molecules or polymeric units to create complex, multi - functional devices. Further the conducting polymers can be synthesized easily by applying a small potential on an electrode using the corresponding monomer, wherein the polymer can also be made neutral by reversing the potential. Therefore, one would be able to reuse the substrate for release of guest molecules particularly the electrode can be reused over a period of five to seven days.

Polymer micelles present a viable alternative, which offer considerable tuning of the micelle size and surface functionality. The present inventors have demonstrated that the anionic surfactants which formed the micelles has modified the surface of oxidised conducting polymer that can be used for "site specific delivery of guest molecules"
Following examples are given by way of illustration.

### Experimental:

### Dynamic Light Scattering study (DLS):

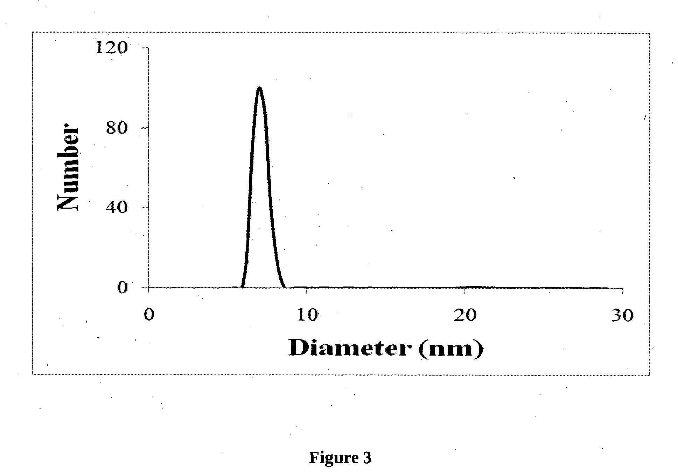

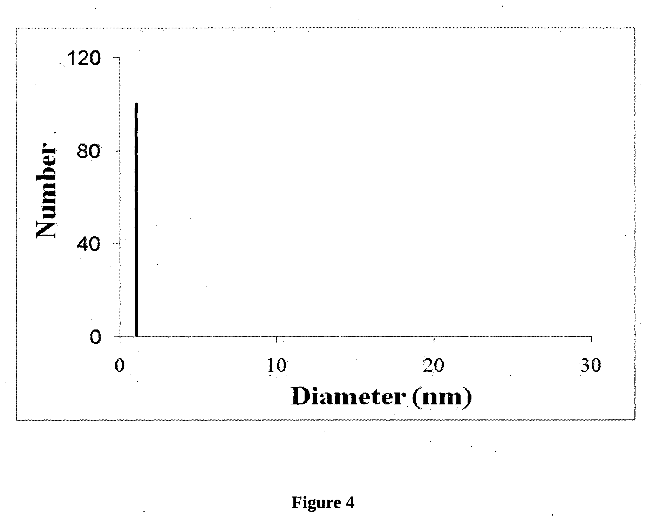

DLS is used to characterize size of various particles including proteins, polymers, micelles, carbohydrates, and nanoparticles wherein the measurement depends on the size of the particle core, the size of surface structures, particle concentration, and type of ions in the medium. The stimuli induced disassembly of nanocontainers is studied using DLS, wherein the 6 mM sodium dodecyl sulfate solution is evaluated which is in the range of 2-20 nm, particularly 6 nm (Fig.3). However on accession of oxidised coducting polymer i.e polyaniline the change in SDS assembly size is determined which is in the range of 0.1-5 nm, particularly 1 nm, essentially indicating the absence of any nanoscale assemblies in the solution (Fig.4).

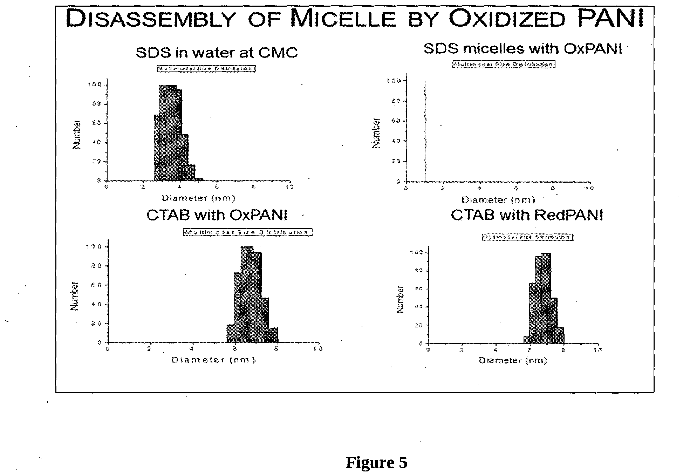

The Fig. 5 indicates comparative assembly size of present SDS micelle with OxPANI and cationic micelles (i.e. CTAB) with reduced and oxidised polyaniline, The present inventors inferred from due experiments that the cationic micelles such as CTAB with reduced and oxidised polyaniline indicates the poor polyvalent interaction, where the assembly size is in the range of 5-8nm.However the SDS in water shows the aggregate size of 2-6 nm at critical micelle concentration.

### UV-Vis Spectroscopy study (UV-vis):

Uv-vis spectroscopy is used to quantify the amount of guest molecule released from disassembly of the SDS micelles upon interaction with conducting polymer.The disassembly and concominant release of sequestered guest molecules are evaluated as function of time, which exhibits multiple emission peaks and ratio of the peak intensity at 372 nm (I₃₇₂) to that at 393 nm (I₃₉₃). It is observed that the guest molecule in SDS assembly corresponds to hydrophobic enviourment which on addition of OxCP to assembly provides hydrophilic enviourment to the encapsulant.This change in the enviourment is due to the disassembly of SDS micelles and concomitant release of cloistered molecule or encapsulant into water.

The present inventors studied the disassembly and concomitant release rate of SDS micelles with different forms of OxCP (Fig.3) wherein the SDS assembly on OxCP nanofibres starts the release of the guest molecule in 15 minutes which is faster by about two times as compared to the bulk OxCP release whereas in presence of NeuCP nanofibres, it shows infinitesimal release, Accordingly the interaction of CTAB micelles with OxCP and NeuCP nanosized fibres are studied , which indicates neither disassembly nor any subsequent release of encapsulated guest molecules.

The invention will now be illustrated with help of examples.

### Example 1:

Sodium dodecyl sulfonate (SDS) was chosen as the ionic micelle to interact with the cationic oxidised conducting polymer (OxCP), Polyaniline (PANI). The surface induced heterogeneous disassembly process was studied using dynamic light scattering (DLS). The aggregate size of 6 mM SDS solution was found to be 6 nm **(****Figure 3****).** To 5 ml SDS solution in water, 100 mg of dry OxCP (PANI) powder was added and these quantities were maintained in all forthcoming experiments. Upon accession of oxidised polyaniline, the change in SDS assembly size was determined to be 1 nm, essentially indicating the absence of any nanoscale assemblies in the solution **(****Figure 4****).**

### Example 2:

This example was generated to determine whether the disassembly is due to the attractive interaction between the positive charges of OxCP and negative charges of the SDS. If so, the assemblies should not break apart in presence of neutral conducting polymer (NeuCP). NeuCP was added to the SDS micelle solution and the assembly size was determined by DLS. The size of the assemblies was found to be 6 nm and remained constant over the period of monitoring. Refer figure 3.

### Example 3: Delivery of molecules using pyrene

The disassembly and concomitant release of sequestered guest molecules were surveyed as a function of time using pyrene, which exhibits multiple emission peaks and the ratio of the peak intensity at 372 nm (I₃₇₂) to that at 393 nm (I₃₉₃). 1 micro molar solution of Pyrene in water was entrapped in SDS assemblies and I₃₇₂/I₃₉₃ was determined to be 0.87, which corresponds to the presence of pyrene in hydrophobic environment. Upon addition of OxCP to the pyrene sequestered SDS assemblies the I₃₇₂/I₃₉₃ started increasing from 0.87 and reached a value of 1.09 in 60 minutes, which indicated that the new environment facing pyrene was hydrophilic. This change in the environment of pyrene is due to the disassembly of SDS micelles and concomitant release of cloistered pyrene into water. The same experiment was carried out using NeuCP and no alteration in I₃₇₂/I₃₉₃ of pyrene was observed, which corroborates that the assemblies is intact in presence of NeuCP.

### Example 4:

UV-vis spectroscopy was used to quantify the amount of pyrene released from the disassembly of the SDS micelles upon interaction with OxCP bulk powder. About 96% of pyrene was released upon the disassembly of nanoscopic containers and the release profile is shown in **Figure 7****.** The unaltered UV-vis spectral features of pyrene indicate that the SDS micelles didn't disassemble and release the guest molecules in presence of NeuCP.

### Example 5: Variation in release kinetics as function of OxCP surface morphology

OxCP nanofibres were synthesized by interfacial polymerization, having diameter about 50 nm (Figure 6). 100 mg nanofibres were then added to the SDS micelle solution and the dimension of the assemblies was determined. The 6 nm assemblies were disassembled to 1 nm, which is roughly the size of the monomer. To survey the rate of the release, these nanosized fibres were added to pyrene encapsulated SDS micelles and I₃₇₂/I₃₉₃ of pyrene was monitored as a function of time. We noted that the disassembly and concomitant release started in 15 minutes, which is faster by about two times as compared to the bulk OxCP release. A control experiment was done using NeuCP nanofibre, which showed infinitesimal release. We also studied the interaction of CTAB micelles with OxCP and NeuCP nanosized fibres, which neither showed any disassembly nor any subsequent release of encapsulated guest molecules. Refer figure 7.

### Example 6:

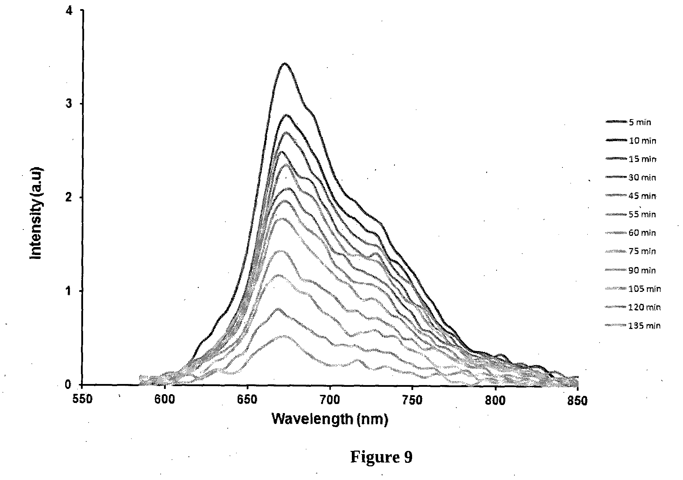

The disassembly and concomitant release of sequestered guest molecules were surveyed as a function of time using nile red. To a 6mMolar solution of SDS in water, 1 micromolar solution of nile red in water was added followed by addition of 100 mg of bulk powder of Poly(3,4-ethylenedioxythiophene). The release of Nile red was studied as a function of time on addition of polymer by monitoring the intensity at 670nm and the result is depicted in figure 9. Decrease in emission intensity as function of time indicates release of guest molecule.

### Example 7:

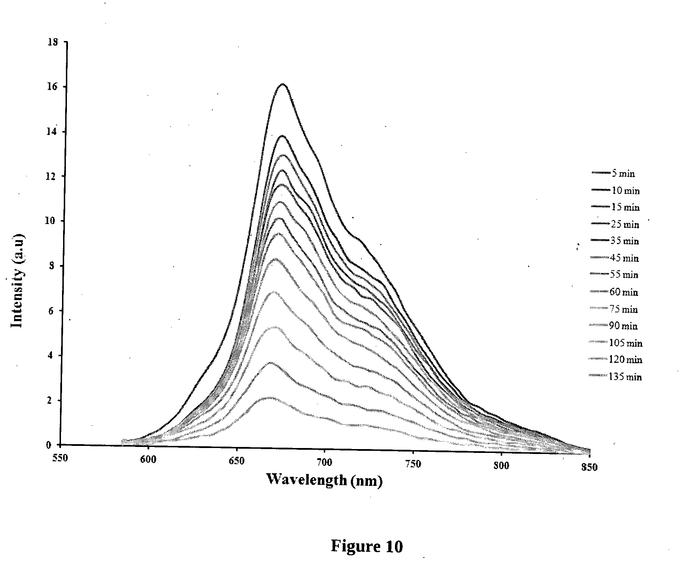

The disassembly and concomitant release of sequestered guest molecules were surveyed as a function of time using nile red. To a 6mMolar solution of SDS in water, 1 micromolar solution of nile red in water was added followed by addition of 100 mg of bulk powder of polyaniline. The release of Nile red was studied as a function of time on addition of polymer by monitoring the intensity at 670nm and the result is depicted in figure 10. Decrease in emission intensity as function of time indicates release of guest molecule.

### Example 8:

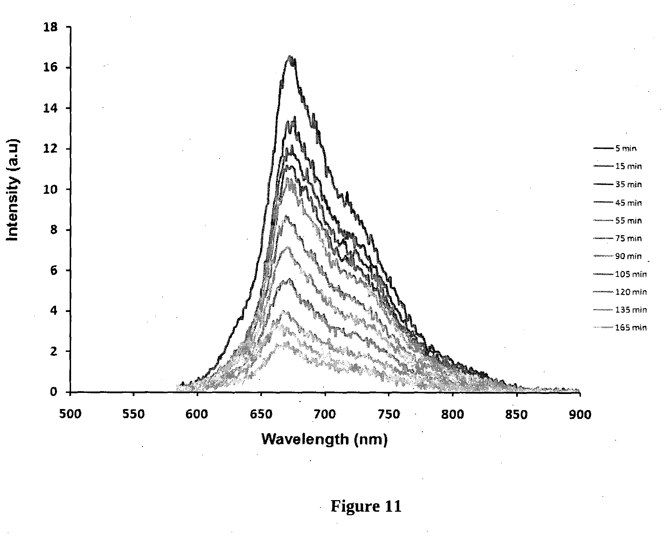

The disassembly and concomitant release of sequestered guest molecules were surveyed as a function of time using nile red. To a 2mMolar solution of DBS in water, 1 micromolar solution of Nile red in water was added followed by addition of 100 mg of bulk powder of polyaniline. The release of nile red was studied as a function of time on addition of polymer by monitoring the intensity at 670nm and the result is depicted in figure 11. Decrease in emission intensity as function of time indicates release of guest molecule.

## Claims

1. A process for preparation of stimuli induced disassembly of nanocontainer comprising of;
a) dissolving anionic surfactant in a solvent at its critical micelle concentration (CMC) to form a solution of anionic micelle;
b) adding a hydrophobic guest molecule to the solution of step (a); and **characterised by**
c) disassembly of the anionic micelle and concomitant release of the hydrophobic guest molecule by addition of a cationic oxidized conducting polymer.

2. The process as claimed in claim 1, wherein the cationic oxidized conducting polymer is selected from polythiophene (PTs), polypyrrole (PPy), Poly(3, 4 ethylenedioxythiophene) and polyaniline (PANI).

3. The process as claimed in claim 1, wherein the cationic oxidized conducting polymer is in bulk, nano, film, patch or device form.

4. The process as claimed in claim 2, wherein the cationic oxidized conducting polymer is polyaniline (PANI).

5. The process as claimed in claim 1, wherein the cationic oxidized conducting polymer is Poly(3, 4 ethylenedioxythiophene).

6. The process as claimed in claim 1, wherein the guest molecule is selected from drugs, dyes, biomolecules and proteins.

7. The process as claimed in claim 1, wherein the anionic surfactant is selected from dodecyl benzene sulphonate (DBS) and sodium dodecyl sulphate (SDS).

## Patentansprüche

1. Verfahren zur Vorbereitung einer Stimuli-induzierten Nanocontainer-Zerlegung, das Folgendes beinhaltet:
a) Auflösen eines anionischen Tensids in einem Lösungsmittel bei seiner kritischen Mizellenkonzentration (CMC), um eine anionische Mizellenlösung zu bilden;
b) Zugeben eines hydrophoben Gastmoleküls zur Lösung von Schritt (a); und **gekennzeichnet durch**
c) Zerlegen der anionischen Mizelle und begleitendes Freisetzen des hydrophoben Gastmoleküls durch Zugabe eines kationischen, oxidierten, leitfähigen Polymers.

2. Verfahren nach Anspruch 1, wobei das kationische, oxidierte, leitfähige Polymer ausgewählt wird aus Polythiophen (PTs), Polypyrrol (PPy), Poly(3, 4 ethylendioxythiophen) und Polyanilin (PANI).

3. Verfahren nach Anspruch 1, wobei das kationische, oxidierte, leitfähige Polymer in Bulk-, Nano-, Film-, Patch- oder Vorrichtungsform vorliegt.

4. Verfahren nach Anspruch 2, wobei das kationische, oxidierte, leitfähige Polymer Polyanilin (PANI) ist.

5. Verfahren nach Anspruch 1, wobei das kationische, oxidierte, leitfähige Polymer Poly(3,4 ethylendioxythiophen) ist.

6. Verfahren nach Anspruch 1, wobei das Gastmolekül ausgewählt wird aus Arzneimitteln, Farbstoffen, Biomolekülen und Proteinen.

7. Verfahren nach Anspruch 1, wobei das anionische Tensid ausgewählt wird aus Dodecylbenzolsulfonat (DBS) und Natriumdodecylsulfat (SDS).

## Revendications

1. Procédé de préparation de dissociation de nanoconteneurs induite par des stimuli, consistant à :
a) dissoudre un tensioactif anionique dans un solvant à sa concentration micellaire critique (CMC) pour former une solution de micelle anionique ;
b) ajouter une molécule hôte hydrophobe à la solution de l'étape (a) ; et **caractérisé par**
c) une dissociation de la micelle anionique et une libération concomitante de la molécule hôte hydrophobe par l'ajout d'un polymère conducteur oxydé cationique.

2. Procédé selon la revendication 1, dans lequel le polymère conducteur oxydé cationique est sélectionné parmi un polythiophène (PTs), un polypyrrole (PPy), un poly(3, 4 éthylènedioxythiophène) et un polyaniline (PANI).

3. Procédé selon la revendication 1, dans lequel le polymère conducteur oxydé cationique est sous forme de vrac, nano, film, patch ou dispositif.

4. Procédé selon la revendication 2, dans lequel le polymère conducteur oxydé cationique est polyaniline (PANI).

5. Procédé selon la revendication 1, dans lequel le polymère conducteur oxydé cationique est poly(3, 4 éthylènedioxythiophène).

6. Procédé selon la revendication 1, dans lequel la molécule hôte est sélectionnée parmi des médicaments, des colorants, des biomolécules et des protéines.

7. Procédé selon la revendication 1, dans lequel le tensioactif anionique est sélectionné parmi le dodécylbenzène sulfonate (DBS) et le dodécylsulfate de sodium (SDS).
